(19) Europäisches Patentamt
European Patent Office
Office européen des brevets

(11) Veröffentlichungsnummer: **0 070 456**
**A1**

# (12) EUROPÄISCHE PATENTANMELDUNG

(21) Anmeldenummer: **82106057.1**

(22) Anmeldetag: **07.07.82**

(51) Int. Cl.³: **C 07 C 147/05**, C 07 C 147/14, A 01 N 41/10 // C07C149/23

(30) Priorität: **18.07.81 DE 3128443**

(43) Veröffentlichungstag der Anmeldung: **26.01.83**
**Patentblatt 83/4**

(84) Benannte Vertragsstaaten: **AT BE CH DE FR GB IT LI NL**

(71) Anmelder: **BAYER AG, Zentralbereich Patente, Marken und Lizenzen, D-5090 Leverkusen 1, Bayerwerk (DE)**

(72) Erfinder: **Lunkenheimer, Winfried, Dr., Bismarckstrasse 29, D-5600 Wuppertal 2 (DE)**
Erfinder: **Brandes, Wilhelm, Dr., Eichendorffstrasse 3, D-5653 Leichlingen (DE)**

(54) **Sulfinyl- und Sulfonyl-acetanilid-Derivate, Verfahren zu ihrer Herstellung sowie ihre Verwendung als Fungizide.**

(57) Sulfinyl- und Sulfonyl-acetanilid-Derivate der allgemeinen Formel I

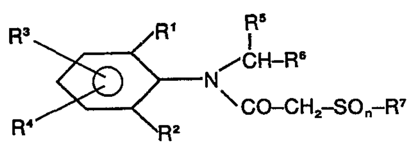

(I),

in welcher

$R^1$ für Alkyl, Alkoxy oder Halogen,

$R^2$ für Wasserstoff, Alkyl, Alkoxy oder Halogen,

$R^3$ und $R^4$ unabhängig voneinander für Wasserstoff, Alkyl oder Halogen,

$R^5$ für Wasserstoff oder Alkyl,

$R^6$ für die Gruppierungen $-CH=C=CH_2$ und $-C\equiv C-R^8$,

$R^7$ für Alkyl, Alkenyl, Alkinyl, Alkoxyalkyl, Aryl oder Aralkyl,

$R^8$ für Wasserstoff oder Halogen,

n für 1 oder 2

mehrere Verfahren zu ihrer Herstellung und ihre Verwendung als Fungizide.

Die neuen Sulfinyl- und Sulfonylacetanilid-Derivate der Formel (I) können aus geeigneten Acetaniliden durch Oxidation oder Umsetzung mit Formaldehyd und Diisopropylamin oder durch Umsetzung von geeigneten Anilinen mit Säurehalogeniden oder -anhydriden hergestellt werden.

EP 0 070 456 A1

0070456

BAYER AKTIENGESELLSCHAFT
Zentralbereich
Patente, Marken und Lizenzen
5090 Leverkusen, Bayerwerk

1 6. 07. 81
Eck/ABc
Ib

# Sulfinyl- und Sulfonyl-acetanilid-Derivate, Verfahren zu ihrer Herstellung sowie ihre Verwendung als Fungizide

Die vorliegende Erfindung betrifft neue Sulfinyl- und Sulfonyl-acetanilid-Derivate, mehrere Verfahren zu ihrer Herstellung und ihre Verwendung als Fungizide.

Es ist bereits bekannt geworden, daß bestimmte N-Allyl- bzw. Allenyl-acetanilid-Derivate mit gutem Erfolg zur Bekämpfung von pilzlichen Pflanzenkrankheiten eingesetzt werden können (DE-OS 29 10 976 und DE-OS 29 16 692). Deren Wirkung ist jedoch bei niedrigen Aufwandmengen und -konzentrationen, insbesondere auch bei der Bekämpfung von Phytophthora-Arten, nicht immer ganz befriedigend.

Es wurden neue Sulfinyl- und Sulfonyl-acetanilid-Derivate der allgemeinen Formel

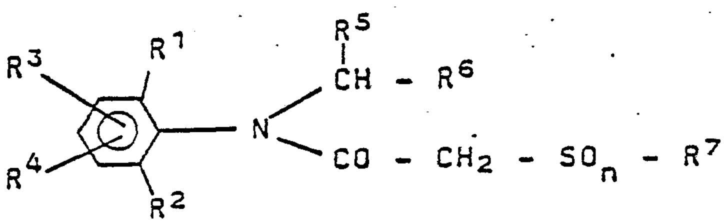

(I),

in welcher

$R^1$ für Alkyl, Alkoxy oder Halogen,

$R^2$ für Wasserstoff, Alkyl, Alkoxy oder Halogen,

$R^3$ und $R^4$ unabhängig voneinander für Wasserstoff, Alkyl oder Halogen,

$R^5$ für Wasserstoff oder Alkyl,

$R^6$ für die Gruppierungen $-CH=C=CH_2$ und $-C\equiv C-R^8$,

$R^7$ für Alkyl, Alkenyl, Alkinyl, Alkoxyalkyl, Aryl oder Aralkyl,

$R^8$ für Wasserstoff oder Halogen,

n für 1 oder 2,

stehen ,

gefunden.

Weiterhin wurde gefunden daß man die Sulfinyl- und Sulfonyl-acetanilid-Derivate der Formel

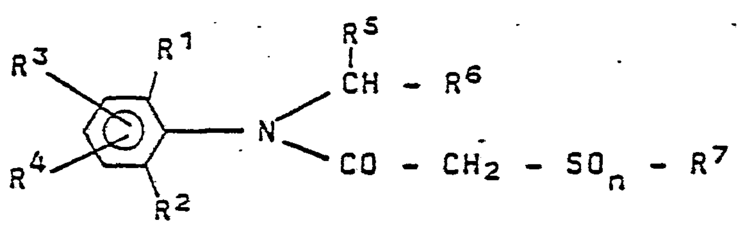

(I),

in welcher

$R^1$ für Alkyl, Alkoxy oder Halogen,

$R^2$ für Wasserstoff, Alkyl, Alkoxy oder Halogen,

$R^3$ und $R^4$ unabhängig voneinander für Wasserstoff, Alkyl oder Halogen,

$R^5$ für Wasserstoff oder Alkyl,

$R^6$ für die Gruppierungen $-CH=CH=CH_2$ und $-C{\equiv}C-R^8$,

$R^7$ für Alkyl, Alkenyl, Alkinyl, Alkoxyalkyl, Aryl oder Aralkyl,

$R^8$ für Wasserstoff oder Halogen,

n für 1 oder 2

stehen,

erhält, wenn man

a) substituierte Acetanilide der Formel

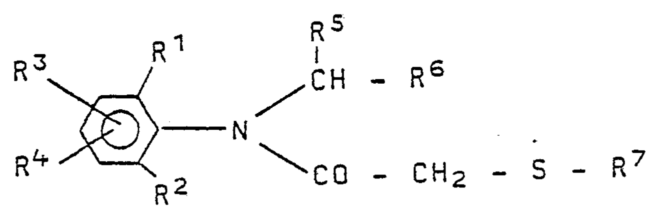

(II),

in welcher

$R^1$ bis $R^7$ die bei Formel I angegebenen Bedeutungen haben,

nach bekannten Methoden in üblicher Weise oxidiert, oder

b) Halogenacetanilide der Formel

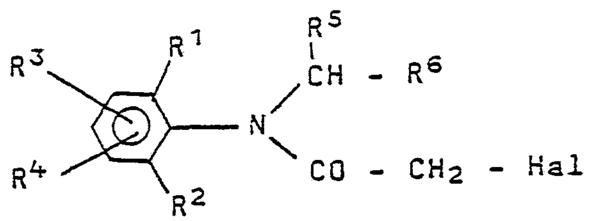

(III),

in welcher
$R^1$ bis $R^6$ die bei Formel I angegebene Bedeutung haben und

Hal für Chlor oder Brom steht,

mit Sulfen- bzw. Sulfinsäure-Derivaten der Formel

$$MSO_n - R^7 \qquad (IV),$$

in welcher
M für Wasserstoff oder ein Alkalimetall steht und

$R^7$ und n die bei Formel I angegebene Bedeutung haben,

in Gegenwart eines Verdünnungsmittels und gegebenenfalls in Gegenwart eines Säurebinders umsetzt, oder

c) Aniline der Formel

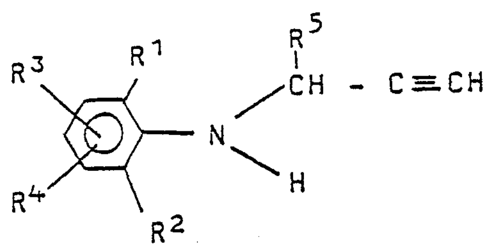

(V),

in welcher

$R^1$ bis $R^5$ die bei Formel I angegebene Bedeutung haben,

mit Säurehalogeniden oder -anhydriden der Formeln

$$Hal\text{-}CO\text{-}CH_2\text{-}SO_n\text{-}R^7 \quad (VIa),$$

oder

$$O(CO\text{-}CH_2\text{-}SO_n\text{-}R^7)_2 \quad (VIb),$$

in welchen

$R^7$ und n die bei Formel I angegebene Bedeutung haben und

Hal für Chlor oder Brom steht,

in Gegenwart eines Verdünnungsmittels und gegebenenfalls in Gegenwart eines Säurebindemittels und/oder Katalysators umsetzt, oder

d) erfindungsgemäße N-Propargyl-acetanilide der Formel

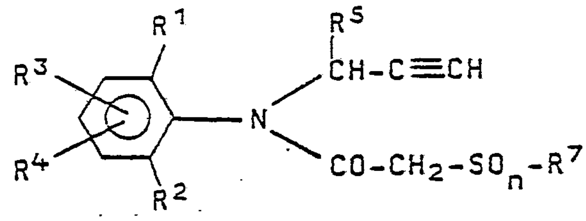

(VII),

in welcher
$R^1$ bis $R^5$, $R^7$ die bei Formel I angegebene Bedeutung haben,

in Gegenwart eines Katalysators mit Formaldehyd und Diisopropylamin umsetzt, oder

e) erfindungsgemäße N-Propargyl-acetanilide der Formel

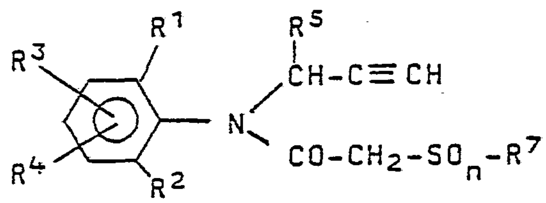

(VII),

in welcher
$R^1$ bis $R^5$, $R^7$ und n die bei Formel I angegebene Bedeutung haben,

mit Alkalihypohalogenit in Gegenwart eines Verdünnungsmittel umsetzt.

Die neuen Sulfinyl- und Sulfonyl-acetanilid-Derivate der Formel (I) weisen starke fungizide Eigenschaften auf. Dabei zeigen überraschenderweise die erfindungsgemäßen Verbindungen eine bessere fungizide Wirkung als die bekannten N-Allyl- bzw. Allenyl-acetanilide (DOS 2 910 976 und DOS 2 916 692).

Die erfindungsgemäßen Sulfinyl- und Sulfonyl-acetanilid-Derivate sind durch die Formel (I) allgemein definiert. In dieser Formel stehen vorzugsweise

$R^1$ für geradkettiges oder verzweigtes Alkyl und Alkoxy mit jeweils 1 bis 4 Kohlenstoffatomen sowie für Fluor, Chlor oder Brom;

$R^2$ für Wasserstoff, geradkettiges oder verzweigtes Alkyl und Alkoxy mit jeweils 1 bis 4 Kohlenstoffatomen sowie für Fluor, Chlor oder Brom;

$R^3$ und $R^4$ unabhängig voneinander für Wasserstoff, geradkettiges oder verzweigtes Alkyl mit 1 bis 4 Kohlenstoffatomen sowie für Fluor, Chlor oder Brom;

$R^5$ für Wasserstoff und geradkettiges oder verzweigtes Alkyl mit 1 bis 4 Kohlenstoffatomen;

$R^6$ für Gruppierungen $-CH=C=CH_2$ und $-C\equiv C-R^8$;

$R^7$ für geradkettiges oder verzweigtes Alkyl mit 1 bis 4 Kohlenstoffatomen, Alkenyl und Alkinyl mit jeweils 2 bis 4 Kohlenstoffatomen, Alkoxyalkyl mit 1 bis 4 Kohlenstoffatomen in jedem Alkylteil, sowie für gegebenenfalls substituiertes Phenyl oder Benzyl, wobei als Substituenten vorzugsweise genannt seien: Halogen, Alkyl mit 1 bis 4 Kohlenstoffatomen, Halogenalkyl mit 1 bis 2 Kohlenstoffatomen und 1 bis 5 gleichen oder verschiedenen Halogenatomen, wie Fluor- und Chloratomen, Alkoxy und Alkylthio mit jeweils 1 bis 2 Kohlenstoffatomen, sowie Nitro und Cyano;

$R^8$ für Wasserstoff, Chlor, Brom oder Iod und

n für 1 oder 2.

Besonders bevorzugt sind diejenigen Verbindungen der Formel (I), in denen

$R^1$ für Methyl, Ethyl, Isopropyl, sek.-Butyl, tert.-Butyl, Methoxy, Ethoxy, Fluor, Chlor oder Brom,

$R^2$ für Wasserstoff, Methyl, Ethyl, Isopropyl, sek.-Butyl, tert.-Butyl, Methoxy, Ethoxy, Fluor, Chlor oder Brom,

$R^3$ und $R^4$ unabhängig voneinander für Wasserstoff, Methyl, Ethyl, Isopropyl, sek.-Butyl, tert.-Butyl, Fluor, Chlor oder Brom,

$R^5$ für Wasserstoff oder Methyl,

$R^6$ für die Gruppierung $-CH{=}C{=}CH_2$ oder $-C{\equiv}C-R^8$,

$R^7$ für Methyl, Ethyl, Isopropyl, sek.-Butyl, tert.-Butyl, Allyl, Propargyl, Methoxymethyl, Ethoxymethyl, 2-Methoxyethyl, Phenyl, 4-Chlorphenyl, 4-Methoxyphenyl oder Benzyl,

$R^8$ für Wasserstoff, Chlor, Brom oder Iod und

n für 1 oder 2

stehen.

Im einzelnen seien außer den bei den Herstellungsbeispielen genannten Verbindungen folgende Verbindungen der allgemeinen Formel (I) genannt:

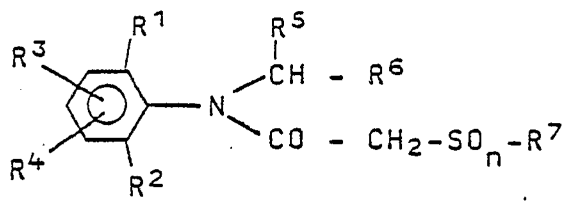

(I)

| $R^1$ | $R^2$ | $R^3$ | $R^4$ | $R^5$ | $R^6$ | n | $R^7$ |
|---|---|---|---|---|---|---|---|
| $CH_3$ | Cl | H | H | $CH_3$ | $-C{\equiv}CH$ | 1 | $CH_3$ |
| $CH_3$ | Cl | H | H | $CH_3$ | $-C{\equiv}CH$ | 2 | $CH_3$ |
| $CH_3$ | Cl | H | H | $CH_3$ | $-CH{=}C{=}CH_2$ | 1 | $CH_3$ |
| $CH_3$ | Cl | H | H | $CH_3$ | $-CH{=}C{=}CH_2$ | 2 | $CH_3$ |
| Cl | Cl | H | H | $CH_3$ | $-C{\equiv}CH$ | 1 | $CH_3$ |
| Cl | Cl | H | H | $CH_3$ | $-C{\equiv}CH$ | 2 | $CH_3$ |
| Cl | Cl | H | H | $CH_3$ | $-CH{=}C{=}CH_2$ | 1 | $CH_3$ |
| Cl | Cl | H | H | $CH_3$ | $-CH{=}C{=}CH_2$ | 2 | $CH_3$ |
| $CH_3$ | $OCH_3$ | H | H | $CH_3$ | $-C{\equiv}CH$ | 1 | $CH_3$ |
| $CH_3$ | $OCH_3$ | H | H | $CH_3$ | $-C{\equiv}CH$ | 2 | $CH_3$ |
| $CH_3$ | $OCH_3$ | H | H | $CH_3$ | $-CH{=}C{=}CH_2$ | 1 | $CH_3$ |

| $R^1$ | $R^2$ | $R^3$ | $R^4$ | $R^5$ | $R^6$ | n | $R^7$ |
|---|---|---|---|---|---|---|---|
| $CH_3$ | $OCH_3$ | H | H | $CH_3$ | $-CH=C=CH_2$ | 2 | $CH_3$ |
| $CH_3$ | $CH_3$ | H | H | H | $-C{\equiv}CH$ | 1 | $CH_3$ |
| $CH_3$ | $CH_3$ | H | H | H | $-C{\equiv}CH$ | 2 | $CH_3$ |
| $CH_3$ | $CH_3$ | H | H | H | $-CH=C=CH_2$ | 1 | $CH_3$ |
| $CH_3$ | $CH_3$ | H | H | H | $-CH=C=CH_2$ | 2 | $CH_3$ |
| $CH_3$ | $CH_3$ | H | H | $CH_3$ | $-C{\equiv}C-I$ | 1 | $CH_3$ |
| $CH_3$ | $CH_3$ | H | H | $CH_3$ | $-C{\equiv}C-I$ | 2 | $CH_3$ |
| $CH_3$ | $CH_3$ | H | H | $CH_3$ | $-C{\equiv}CH$ | 1 | $C_2H_5$ |
| $CH_3$ | $CH_3$ | H | H | $CH_3$ | $-C{\equiv}CH$ | 2 | $C_2H_5$ |
| $CH_3$ | $CH_3$ | H | H | $CH_3$ | $-CH=C=CH_2$ | 1 | $C_2H_5$ |
| $CH_3$ | $CH_3$ | H | H | $CH_3$ | $-CH=C=CH_2$ | 2 | $C_2H_5$ |
| $CH_3$ | $CH_3$ | H | H | $CH_3$ | $-C{\equiv}CH$ | 1 | $-CH_2-CH=CH_2$ |
| $CH_3$ | $CH_3$ | H | H | $CH_3$ | $-C{\equiv}CH$ | 2 | $-CH_2-CH=CH_2$ |
| $CH_3$ | $CH_3$ | H | H | $CH_3$ | $-CH=C=CH_2$ | 1 | $-CH_2-CH=CH_2$ |
| $CH_3$ | $CH_3$ | H | H | $CH_3$ | $-CH=C=CH_2$ | 2 | $-CH_2-CH=CH_2$ |
| $CH_3$ | $CH_3$ | H | H | $CH_3$ | $-C{\equiv}CH$ | 1 | $-CH_2-C{\equiv}CH$ |
| $CH_3$ | $CH_3$ | H | H | $CH_3$ | $-C{\equiv}CH$ | 2 | $-CH_2-C{\equiv}CH$ |
| $CH_3$ | $CH_3$ | H | H | $CH_3$ | $-CH=C=CH_2$ | 1 | $-CH_2-C{\equiv}CH$ |
| $CH_3$ | $CH_3$ | H | H | $CH_3$ | $-CH=C=CH_2$ | 2 | $-CH_2-C{\equiv}CH$ |
| $CH_3$ | $CH_3$ | H | H | $CH_3$ | $-C{\equiv}CH$ | 1 | 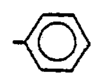 |
| $CH_3$ | $CH_3$ | H | H | $CH_3$ | $-C{\equiv}CH$ | 2 | 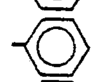 |
| $CH_3$ | $CH_3$ | H | H | $CH_3$ | $-CH=C=CH_2$ | 1 | 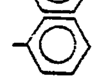 |
| $CH_3$ | $CH_3$ | H | H | $CH_3$ | $-CH=C=CH_2$ | 1 | 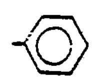 |
| $CH_3$ | $CH_3$ | H | H | $CH_3$ | $-C{\equiv}CH$ | 1 | $-CH_2-$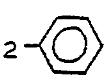 |
| $CH_3$ | $CH_3$ | H | H | $CH_3$ | $-C{\equiv}CH$ | 2 | $-CH_2-$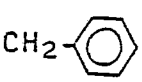 |
| $CH_3$ | $CH_3$ | H | H | $CH_3$ | $-CH=C=CH_2$ | 1 | $-CH_2-$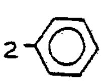 |
| $CH_3$ | $CH_3$ | H | H | $CH_3$ | $-CH=C=CH_2$ | 2 | $-CH_2-$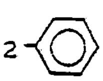 |

Verwendet man beispielsweise 2',6'-Dimethyl-N-(1-methyl-2,3-butadienyl)-2-methylthioacetanilid und m-Chlorperbenzoesäure als Ausgangsstoffe, so kann der Reaktionsablauf durch das folgende Formelschema wiedergegeben werden (Verfahren a):

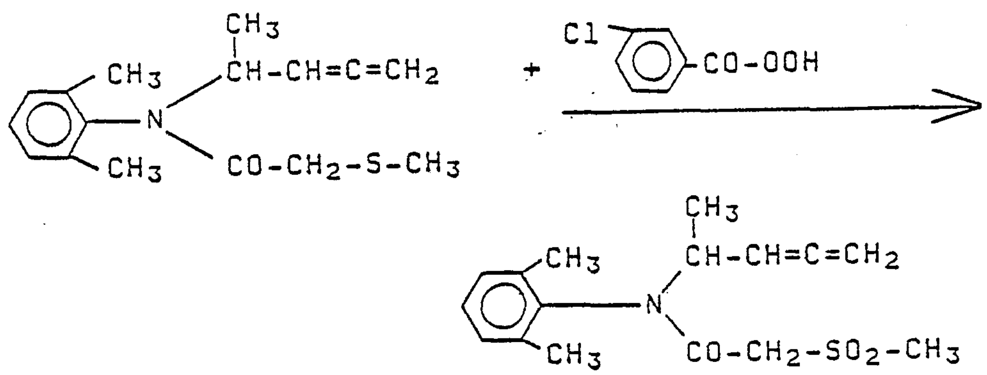

Verwendet man beispielsweise 2',6'-Dimethyl-N-propargyl-2-chloracetanilid und Methylsulfinsäure als Ausgangsstoffe, so kann der Reaktionsablauf durch das folgende Formelschema wiedergegeben werden (Verfahren b):

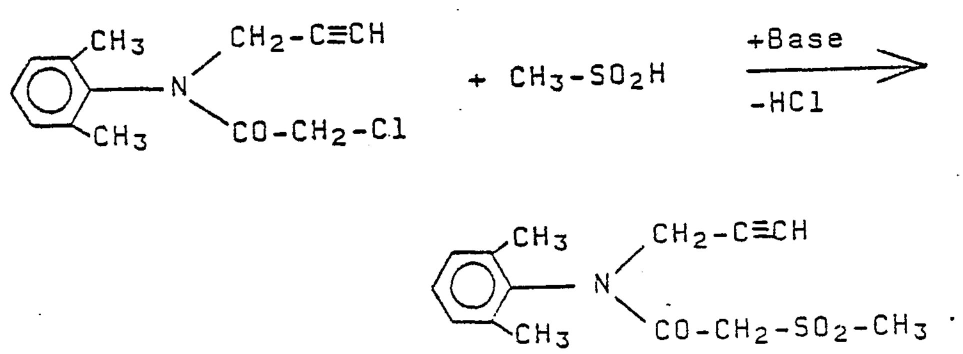


Verwendet man beispielsweise 2',6'-Dimethyl-N-(1-methyl-2-propinyl)-anilin und Methylsulfonylacetylchlorid als

Ausgangsstoffe, so kann der Reaktionsablauf durch das folgende Formelschema wiedergegeben werden (Verfahren c):

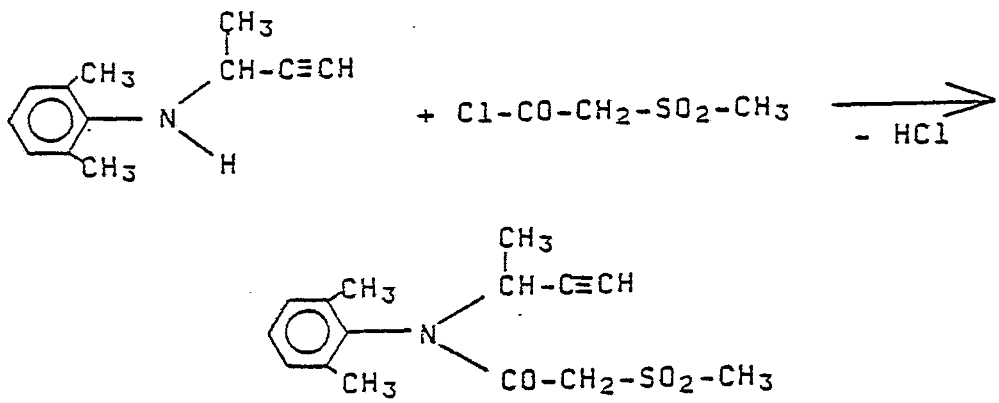


Verwendet man beispielsweise 2',6'-Dimethyl-N-(1-methyl-2-propinyl)-2-methylsulfonylacetanilid, Paraformaldehyd und Diisopropylamin als Ausgangsstoffe und Kupfer-(I)-bromid als Katalysator, so kann der Reaktionsablauf durch das folgende Formelschema wiedergegeben werden (Verfahren d):

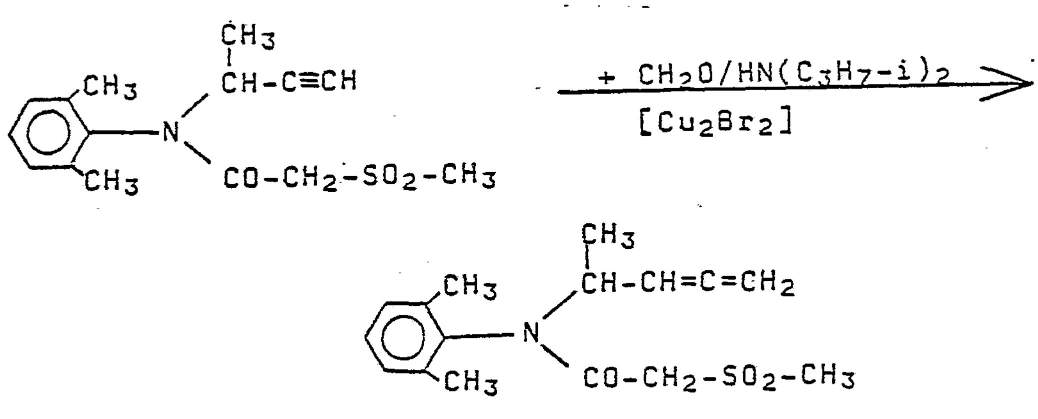


Verwendet man beispielsweise 2',6'-Dimethyl-N-(1-methyl-2-propinyl)-2-methyl-sulfonylacetanilid und Kaliumhypo-

bromit als Ausgangsstoffe, so kann der Reaktionsablauf durch das folgende Formelschema wiedergegeben werden (Verfahren e):

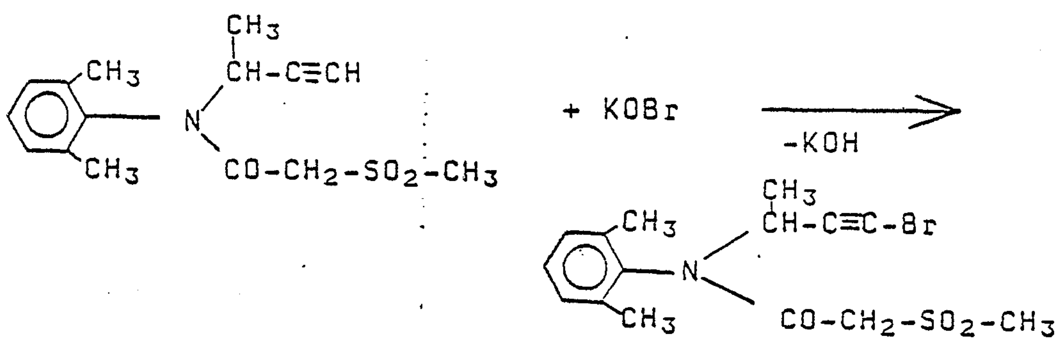


Die für die Durchführung des erfindungsgemäßen Verfahrens (a) als Ausgangsstoffe benötigten substituierten Acetanilide sind durch die Formel (II) allgemein definiert. In dieser Formel stehen $R^1$ bis $R^7$ vorzugsweise für diejenigen Reste, die bereits im Zusammenhang mit der Beschreibung der erfindungsgemäßen Stoffe der Formel (I) vorzugsweise für diese Substituenten genannt wurden.

Die substituierten Acetanilide der Formel (II) sind teilweise bekannt (DE-OS 28 47 287), teilweise sind sie Gegenstand einer eigenen älteren Anmeldung, die noch nicht veröffentlicht ist (Deutsche Patentanmeldung P 30 27 758 vom 22.7.1980). Im erfindungsgemäßen Verfahren einsetzbare Acetanilide dieser Patentanmeldung können nach dem unter d) beschriebenen Verfahren aus geeigneten Ausgangssubstanzen hergestellt werden.

Die erfindungsgemäße Oxidation gemäß Verfahren (a) erfolgt durch die Umsetzung mit üblichen anorganischen oder organischen Oxidationsmitteln. Hierzu gehören vorzugsweise organische Persäuren, wie z.B. Peressigsäure, p-Nitroperbenzoesäure, m-Chlorperbenzoesäure; anorganische Persäuren, wie z.B. Periodsäure; weiterhin Wasserstoffperoxid in Eisessig oder Methanol.

Die für die Durchführung des erfindungsgemäßen Verfahrens (b) als Ausgangsstoffe benötigten Halogenacetanilide sind durch die Formel (III) allgemein definiert. In dieser Formel stehen $R^1$ bis $R^6$ vorzugsweise für diejenigen Reste, die bereits im Zusammenhang mit der Beschreibung der erfindungsgemäßen Stoffe der Formel (I) vorzugsweise für diese Substituenten genannt wurden.

Die Halogenacetanilide der Formel (III) sind teilweise bekannt (vgl. z.B. US-Patentschrift 4 001 325 und DE-OS 29 19 196), teilweise sind sie Gegenstand einer eigenen älteren Anmeldung, die noch nicht veröffentlicht ist (Deutsche Patentanmeldung P 30 12 623 vom 1.4.1980); bzw. können sie in allgemein üblicher Art und Weise erhalten werden.

Die außerdem für das erfindungsgemäße Verfahren (b) als Ausgangsstoffe benötigten Sulfen- bzw. Sulfinsäure-Derivate sind durch die Formel (IV) allgemein definiert. In dieser Formel stehen $R^7$ und der Index n vorzugsweise für die Bedeutungen, die bereits im Zusammenhang mit der Beschreibung der erfindungsgemäßen Stoffe der Formel (I)

vorzugsweise genannt wurden. M steht vorzugsweise für Wasserstoff, Natrium und Kalium.

Die Sulfen- bzw. Sulfinsäure-Derivate der Formel (IV) sind bekannt, bzw. können sie in allgemein üblicher Art und Weise erhalten werden.

Die für die Durchführung des erfindungsgemäßen Verfahrens (c) als Ausgangsstoffe benötigten Aniline sind durch die Formel (V) allgemein definiert. In dieser Formel stehen $R^1$ bis $R^5$ vorzugsweise für diejenigen Reste, die bereits im Zusammenhang mit der Beschreibung der erfindungsgemäßen Stoffe der Formel (I) vorzugsweise für diese Substituenten genannt wurden.

Die Aniline der Formel (V) sind bekannt (US-Patentschriften 3 535 377 und 4 001 325 sowie DE-OS 28 47 287), bzw. können sie nach bekannten Verfahren erhalten werden.

Die außerdem für das erfindungsgemäße Verfahren (c) als Ausgangsstoffe zu verwendenden Säurehalogenide bzw. -anhydride sind durch die Formeln (VIa) und (VIb) allgemein definiert. In diesen Formeln stehen $R^7$ und der Index n vorzugsweise für die Bedeutungen, die bereits im Zusammenhang mit der Beschreibung der erfindungsgemäßen Stoffe der Formel (I) vorzugsweise genannt wurden.

Die Säurehalogenide bzw. -anhydride der Formeln (VIa) und (VIb) sind bekannte Verbindungen der organischen Chemie oder können nach bekannten Verfahren hergestellt werden.

Die für die Durchführung der erfindungsgemäßen Verfahren

(d) und (e) als Ausgangsstoffe benötigten N-Propargylacetanilide sind durch die Formel (VII) allgemein definiert. In dieser Formel stehen $R^1$ bis $R^5$, $R^7$ und der Index n vorzugsweise für die Bedeutungen, die bereits im Zusammenhang mit der Beschreibung der erfindungsgemäßen Stoffe der Formel (I) vorzugsweise genannt wurden.

Die N-Propargyl-acetanilide der Formel (VII) sind erfindungsgemäße Verbindungen.

Die außerdem für das erfindungsgemäße Verfahren (d) als Ausgangsstoffe benötigten Verbindungen Formaldehyd (in Form von Paraformaldehyd oder Trioxan) und Diisopropylamin sind allgemein bekannte Verbindungen der organischen chemie, ebenso die außerdem für das erfindungsgemäße Verfahren (e) als Ausgangsstoffe benötigten Alkalihypohalogenite, wie z.B. Natrium- und Kalium-hypochlorit, -bromit oder -iodit.

Als Verdünnungsmittel kommen für die erfindungsgemäße Umsetzung gemäß Verfahren (a) vorzugsweise unter den Reaktionsbedingungen inerte organische Lösungsmittel in Frage, beispielsweise Ketone, wie Diethylketon, insbesondere Aceton und Methylisobutylketon; Nitrile, wie Propionitril, insbesondere Acetonitril, Ether, wie Tetrahydrofuran oder Dioxan; aliphatische und aromatische Kohlenwasserstoffe, wie Petrolether, Benzol, Toluol oder Xylol; halogenierte Kohlenwasserstoffe, wie Methylenchlorid, Tetrachlorkohlenstoff, Chloroform oder Chlorbenzol, Ester, wie Essigester, Alkohole, wie Methanol und Carbonsäuren, wie Essigsäure.

Die Reaktionstemperaturen können bei der Durchführung der Oxidation gemäß Verfahren (a) in einem bestimmten Bereich variiert werden. Im allgemeinen arbeitet man von -20°C bis 40°C, vorzugsweise von -10 bis 30°C.

Bei der Durchführung der erfindungsgemäßen Oxidation gemäß Verfahren (a) setzt man auf 1 Mol der Verbindungen der Formel (II) 1 oder 2 Mol Oxidationsmittel ein, je nach dem ob durch Oxidation des sulfidischen Schwefels die Sulfinyl- (n=1) oder Sulfonyl-acetanilid-Derivate (n=2) der Formel I hergestellt werden sollen.

Bei der Anwendung von 1 Mol Oxidationsmittel, beispielsweise 1 mol m-Chlorperbenzoesäure in Methylenchlorid bei Temperaturen von -20 bis +20°C, entstehen vorzugsweise die erfindungsgemäßen Verbindungen der Formel (I) mit n=1.

Mit 2 Mol Oxidationsmittel und höheren Temperaturen (0 bis 40°C) entstehen vorzugsweise die erfindungsgemäßen Verbindungen der Formel (I) mit n=2.

Die Isolierung der Oxidationsprodukte erfolgt in üblicher Weise.

Als Verdünnungsmittel für die erfindungsgemäße Umsetzung gemäß Verfahren (b) kommen vorzugsweise unter den Reaktionsbedingungen inerte organische Lösungsmittel in Frage, beispielsweise Ketone, wie Diethylketon, insbesondere Aceton und Methylisobutylketon; Nitrile, wie Propionitril, insbesondere Acetonitril; Ether, wie Tetrahydrofuran oder Dioxan; aliphatische und aromatische Kohlenwasserstoffe, wie Petrolether, Benzol, Toluol oder Xylol; halogenierte Kohlenwasserstoffe, wie Methylenchlorid, Tetrachlorkohlenstoff, Chloroform oder Chlorbenzol und Ester, wie Essigester.

Die Umsetzung nach Verfahren (b) kann gegebenenfalls in Gegenwart eines Säurebinders durchgeführt werden. Man kann alle üblicherweise verwendbaren anorganischen oder organischen Säurebinder zugeben, beispielsweise Alkalicarbonate, wie Natriumcarbonat, Kaliumcarbonat und Natriumhydrogencarbonat, oder niedere tertiäre Alkylamine, Cycloalkylamine oder Aralkylamine, wie Triethylamin, Dimethylbenzylamin, oder Pyridin und Diazabicyclooctan.

Die Reaktionstemperaturen können beim Verfahren (b) in einem größeren Bereich variiert werden. Im allgemeinen arbeitet man von 20°C bis 150°C, vorzugsweise von 60°C bis 120°C.

Bei der Durchführung des erfindungsgemäßen Verfahrens (b) setzt man auf 1 Mol der Verbindungen der Formel (III) vorzugsweise etwa 1 bis 2 Mol der Verbindungen der Formel (IV) und gegebenenfalls etwa 1 bis 2 Mol Säurebinder ein. Die Isolierung der Verbindungen der Formel (I) erfolgt in üblicher Weise.

Als Verdünnungsmittel kommen für die erfindungsgemäße Umsetzung gemäß Verfahren (c) vorzugsweise unter Reaktionsbedingungen inerte organische Lösungsmittel in Frage. Hierzu gehören vorzugsweise organische Lösungsmittel, beispielsweise Ketone, wie Diethylketon, insbesondere Aceton und Methylisobutylketon; Nitrile, wie Propionitril, insbesondere Acetonitril, Ether, wie Tetrahydrofuran oder Dixoan; aliphatische und aromatische Kohlenwasserstoffe, wie Petrolether, Benzol, Toluol oder Xylol; halogenierte Kohlenwasserstoffe, wie Methylenchlorid, Tetrachlorkohlenstoff, Chloroform oder Chlorbenzol und Ester, wie Essigester.

Das erfindungsgemäße Verfahren (c) kann gegebenenfalls in Gegenwart von Säurebindern durchgeführt werden. Als solche können übliche Säurebindemittel verwendet werden. Hierzu gehören vorzugsweise organische Basen, beispielsweise tertiäre Amine, wie Triethylamin, oder wie Pyridin, ferner anorganische Basen, wie Alkalihydroxide und Alkalicarbonate.

Das erfindungsgemäße Verfahren (c) kann in Gegenwart eines Katalysators ausgeführt werden. Als Katalysator können beispielsweise Amide von organischen Säuren wie Dimethylformamid eingesetzt werden.

Die Reaktionstemperaturen können bei der Durchführung des erfindungsgemäßen Verfahrens (c) in einem größeren Bereich variiert werden. Im allgemeinen arbeitet man von 0 bis 100°C.

Bei der Durchführung des erfindungsgemäßen Verfahrens (c) werden die Reaktionspartner vorzugsweise in etwa molaren Mengen eingesetzt. Die Isolierung der Verbindungen der Formel (I) erfolgt in üblicher Weise.

Als Verdünnungsmittel kommen für die erfindungsgemäße Umsetzung gemäß Verfahren (d) vorzugsweise unter den Reaktionsbedingungen inerte organische Lösungsmittel in Frage. Hierzu gehören vorzugsweise Ether, beispielsweise Tetrahydrofuran oder Dioxan.

Als Katalysatoren kommen für das Verfahren (d) vorzugsweise Metallhalogenide, beispielsweise Kupfer(I)-halogenide, wie Kupfer(I)-bromid oder -chlorid in Frage.

Die Reaktionstemperaturen können bei der Durchführung des erfindungsgemäßen Verfahrens (d) in einem größeren Bereich variiert werden. Im allgemeinen arbeitet man von 60 bis 120°C.

Bei der Durchführung des erfindungsgemäßen Verfahrens (d) setzt man vorzugsweise auf 1 Mol der Verbindungen der Formel (VII) etwa 1 bis 2 Mol Formaldehyd, etwa 1 bis 1,5 Mol Diisopropylamin und etwa 0,1 bis 0,5 Mol Katalysator ein. Die Isolierung der Umsetzungsprodukte erfolgt nach üblichen Methoden(vergleiche auch die Angaben in J.Chem.Soc.Comm. 1979, 859; Tetrahedron Letters 21, 929 (1980)).

Als Verdünnungsmittel kommen für die erfindungsgemäße Umsetzung gemäß Verfahren (e) Wasser sowie unter den Reaktionsbedingungen inerte organische Lösungsmittel in Frage. Hierzu gehören vorzugsweise Alkohole, wie Methanol oder Ethanol, Ether wie Diethylether, Dioxan oder Tetrahydrofuran.

In besonderen Fällen kann es vorteilhaft sein in einem Zweiphasengemisch, beispielsweise einem Gemisch aus Ether/ Wasser die Umsetzung auszuführen.

Die Reaktionstemperaturen können beim Verfahren (e) in einem größeren Bereich variiert werden. Im allgemeinen

arbeitet man von 0 bis 100°C, vorzugsweise von 0 bis 40°C.

Bei der Durchführung des erfindungsgemäßen Verfahrens (e) setzt man vorzugsweise auf 1 Mol der Verbindung der Formel (VII) etwa 1 bis 1,5 Mol Hypohalogenid ein, wobei das Alkalihypohalogenit in-situ aus dem entsprechenden Halogen und dem Alkalihydroxid erzeugt werden kann (Houben-Weyl, Bd. V/2a, S. 608-610 (1977)).

Mögliche Enantiomere der erfindungsgemäßen Verbindungen werden von Formel (I) ebenfalls umfaßt.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Als Pflanzenschutzmittel können die erfindungsgemäßen Wirkstoffe mit besonders gutem Erfolg zur Bekämpfung von Oomyceten, z.B. gegen den Erreger der Kraut- und Braunfäule der Tomate und Kartoffel (Phytophthora infestans) eingesetzt werden. Besonders hervorzuheben ist,

daß die erfindungsgemäßen Wirkstoffe nicht nur eine protektive, sondern auch eine systemische Wirkung entfalten. So gelingt es, Pflanzen gegen Pilzbefall zu schützen, wenn man den Wirkstoff über den Boden und die Wurzel oder über das Saatgut den oberirdischen Teilen der Pflanze zuführt.

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsionskonzentrate, Saatgutpuder, Wirkstoff-imprägnierte Natur- und synthetische Stoffe, Feinverkapselungen in polymeren Stoffen und in Hüllmassen für Saatgut, ferner in Formulierungen mit Brennsätzen, wie Räucherpatronen, -dosen, -spiralen u.ä. sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie

Butanol oder Glycol sowie deren Ether und Ester, Ketone, wie Aceton, Methylethylketon, Methylisobutylketon oder Cyclohexanon, starke polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material, wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglykolether, Alkylsulfonate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie

Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 1 und 95 Gew.-% Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 Gew.-%, am Wirkungsort erforderlich.

Herstellungsbeispiele

Beispiel 1

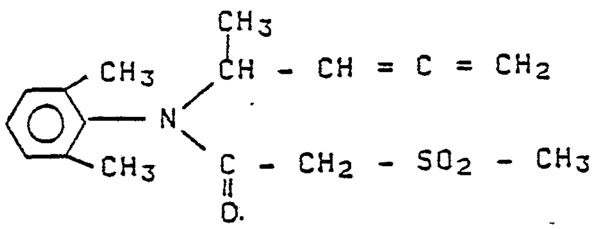

(Verfahren a)

In eine Lösung von 3,2 g (11,6 mmol) 2',6'-Dimethyl-N-(1-methyl-2,3-butadienyl)-2-methylthioacetanilid in 30 ml Dichlormethan wird bei 0°C eine Lösung von 4,72 g (23,2 mmol) 85 %iger 3-Chlorperbenzoesäure in 70 ml Dichlormethan getropft und die Mischung 1 Stunde bei 0°C und 3 Stunden bei 20°C gerührt. Man wäscht die Reaktionslösung dreimal mit je 80 ml 10 %iger Natriumcarbonat-Lösung, zweimal mit je 200 ml Wasser, trocknet über Natriumsulfat und dampft im Vakuum ein. Man erhält so 3,4 g 2',6'-Dimethyl-N-(1-methyl-2,3-butadienyl)-2-methylsulfonyl-acetanilid als hellbraunes Öl mit einem Brechungsindex $n_D^{20}$ = 1,5352.

Herstellung des Ausgangsproduktes

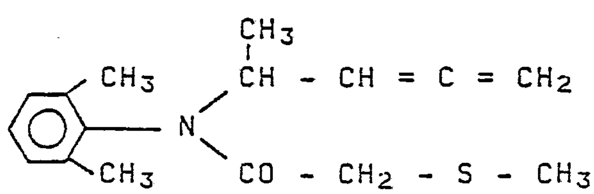

Eine Mischung aus 52,2 g (0,2 mol) 2',6'-Dimethyl-N-(1-methyl-2-propinyl)-2-methylthio-acetanilid, 9,6 g

(0,32 mol) Paraformaldeyhd, 24,3 g (0,24 mol) Diisopropylamin, 9,5 g (0,066 mol) Kupfer(I)-bromid und 200 ml Dioxan wird 21 Stunden unter Rückfluß erhitzt. Man filtriert, destilliert das Dioxan im Vakuum ab, löst den Rückstand in 500 ml Essigsäure, wäscht mit 600 ml 1 M Natriumcyanid-Lösung, wäscht mit je 300 ml Wasser, 1 M Citronensäure, gesättigter Natriumhydrogencarbonat-Lösung und wieder Wasser, trocknet über Natriumsulfat und dampft die Lösung ein. Den öligen Rückstand chromatographiert man an einer Kieselgel-Säule (5 x 100 cm) mit 0-20 %igem Essigester/Toluol. Man erhält so 19,6 g (35,6 % der Theorie) 2',6'-Dimethyl-N-(1-methyl-2,3-butadienyl)-2-methylthio-acetanilid als orangefarbenes Öl.

Beispiel 2

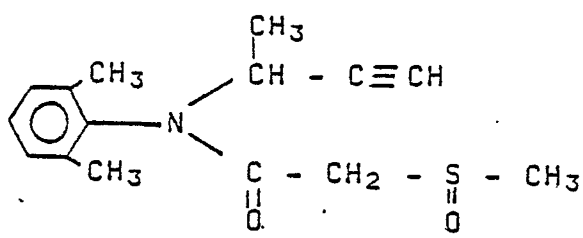

(Verfahren a)

In eine Lösung von 24,7 g (0,095 mol) 2',6'-Dimethyl-N-(1-methyl-2-propinyl)-2-methylthio-acetanilid in 150 ml Dichlormethan tropft man bei 0°C eine Lösung von 19,3 g (0,035 mol) 85 %iger 3-Chlorperbenzoesäure in 350 ml Dichlormethan und rührt 2 Stunden bei 0°C. Man läßt die Reaktionsmischung in 40 Minuten auf 15°C kommen, wäscht mit gesättigter Natriumhydrogencarbonat-Lösung (3 x

200 ml) und Wasser (2 x 400 ml), trocknet über Natriumsulfat und dampft ein. Der Rückstand wird mit Petrolether/Ether (6 : 1) verrieben. Man erhält so 20,6 g (78,3 % der Theorie) 2',6'-Dimethyl-N-(1-methyl-2-propinyl)-2-methylsulfinyl-acetanilid vom Schmelzpunkt 71-73°C.

Herstellung des Ausgangsproduktes

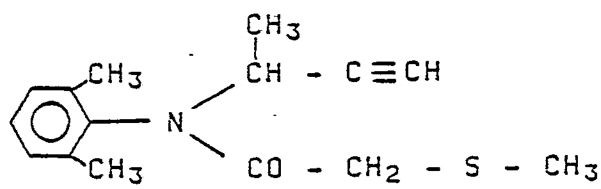

In eine unter Rückfluß siedende Lösung von 9,0 g (0,05 mol) 2',6'-Dimethyl-N-(1-methyl-2-propinyl)-anilin und 5,95 g (0,075 mol) Pyridin in 50 ml Tetrahydrofuran tropft man 9,35 g (0,075 mol) Methylthio-acetylchlorid und erhitzt 2,5 Stunden unter Rückfluß. Nach Abdestillieren des Lösungsmittels wird der Rückstand zwischen 100 ml Essigester und 50 ml verdünnter Salzsäure verteilt, die organische Phase mit verdünnter Salzsäure (50 ml), 5 %iger Natronlauge (100 ml) und Wasser (2 x 150 ml) gewaschen, über Natriumsulfat getrocknet und eingedampft. Der Rückstand wird in 50 ml Acetonitril mit A-Kohle behandelt. Nach Filtrieren und Eindampfen erhält man 11,5 g (88,1 % der Theorie)

2',6'-Dimethyl-N-(1-methyl-2-propinyl)-2-methylthio-acet-anilid als rotbraunes Öl vom Brechungsindex $n_D^{20}$ = 1,5470.

In entsprechender Weise und gemäß den angegebenen Verfahren werden die folgenden Verbindungen der allgemeinen Formel

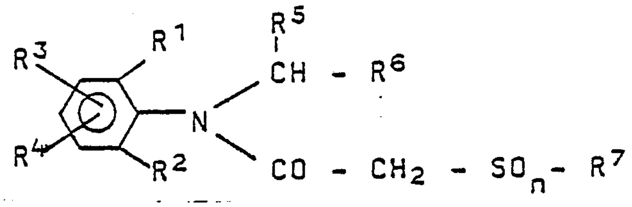

(I)

erhalten:

| Bsp. Nr. | $R^1$ | $R^2$ | $R^3$ | $R^4$ | $R^5$ | $R^6$ | n | $R^7$ | Physik.-konstante |
|---|---|---|---|---|---|---|---|---|---|
| 3 | $CH_3$ | $CH_3$ | H | H | $CH_3$ | $-C\equiv CH$ | 2 | $CH_3$ | Schmp. 88-89°C |
| 4 | $CH_3$ | $CH_3$ | H | H | $CH_3$ | $-CH=C=CH_2$ | 1 | $CH_3$ | $n_D^{20}$ = 1,5533 |
| 5 | $CH_3$ | $CH_3$ | H | H | $CH_3$ | $-C\equiv CH$ | 1 | 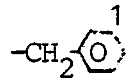 | Schmp. 87-89°C |
| 6 | $CH_3$ | $CH_3$ | H | H | $CH_3$ | $-C\equiv CH$ | 2 | 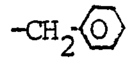 | $n_D^{20}$= 1,5562 |
| 7 | $CH_3$ | $CH_3$ | H | H | $CH_3$ | $-CH=C=CH_2$ | 2 | 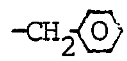 | $n_D^{20}$= 1,5693 |

Anwendungsbeispiele

In den nachfolgenden Beispielen werden die nachstehend angegebenen Verbindungen als Vergleichssubstanzen eingesetzt:

(A) 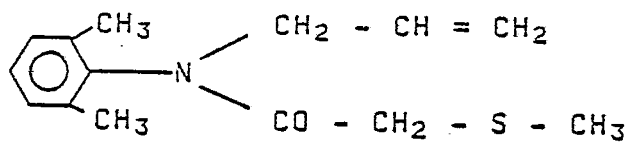

(B) 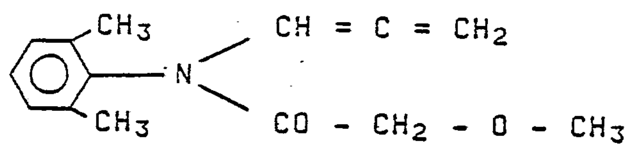

(C) 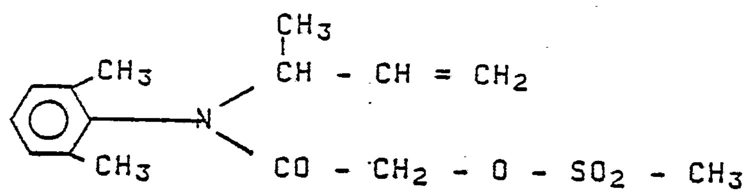

Beispiel A

Phytophthora-Test (Tomate) / protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Sporensuspension von Phytophthora infestans inokuliert.

Die Pflanzen werden in einer Inkubationskabine mit 100 % relativer Luftfeuchtigkeit und ca. 20°C aufgestellt.

3 Tage nach der Inokulation erfolgt die Auswertung.

Eine deutliche Überlegenheit in der Wirksamkeit gegenüber dem Stand der Technik zeigen in diesem Test z.B. die Verbindungen gemäß folgender Herstellungsbeispiele: 1,2 und 3.

Beispiel B

Phytophthora-Test (Tomate) / systemisch

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf systemische Eigenschaften wird die Wirkstoffzubereitung auf Einheitserde gegossen, in der sich junge versuchsbereite Pflanzen befinden. 3 Tage nach der Behandlung werden die Pflanzen mit einer wäßrigen Sporensuspension von Phytophthora infestans inokuliert.

Die Pflanzen werden in einer Inkubationskabine mit 100 % relativer Luftfeuchtigkeit und ca. 20°C aufgestellt.

3 Tage nach der Inokulation erfolgt die Auswertung.

Eine deutliche Überlegenheit in der Wirksamkeit gegenüber dem Stand der Technik zeigen in diesem Test z.B. die Verbindungen gemäß folgender Herstellungsbeispiele: 1, 2 und 3.

Patentansprüche

1. Sulfinyl- und Sulfonyl-acetanilid-Derivate der allgemeinen Formel

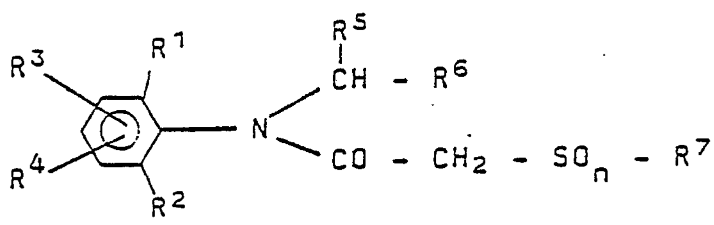

(I)

in welcher

$R^1$ für Alkyl, Alkoxy oder Halogen,

$R^2$ für Wasserstoff, Alkyl, Alkoxy oder Halogen,

$R^3$ und $R^4$ unabhängig voneinander für Wasserstoff, Alkyl oder Halogen,

$R^5$ für Wasserstoff oder Alkyl,

$R^6$ für die Gruppierungen $-CH=C=CH_2$ und $-C\equiv C-R^8$,

$R^7$ für Alkyl, Alkenyl, Alkinyl, Alkoxyalkyl, Aryl oder Aralkyl,

$R^8$ für Wasserstoff oder Halogen,

n für 1 oder 2

steht.

2. Sulfinyl- und Sulfonyl-acetanilid-Derivate der allgemeinen Formel

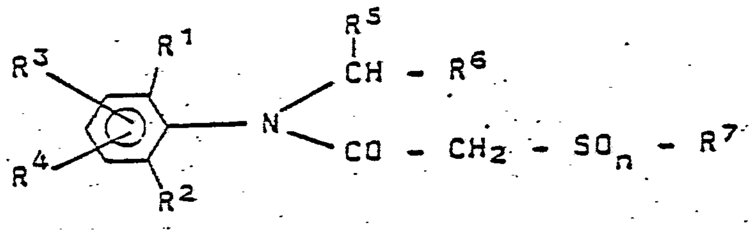

(I)

in welcher

$R^1$ für Alkyl oder Alkoxy mit 1 bis 4 C-Atomen oder Fluor, Chlor oder Brom,

$R^2$ für Wasserstoff, Alkyl oder Alkoxy mit 1 bis 4 C-Atomen oder Fluor, Chlor oder Brom,

$R^3$ und $R^4$ unabhängig voneinander für Wasserstoff, Alkyl mit 1 bis 4 C-Atomen oder Fluor, Chlor oder Brom,

$R^5$ für Wasserstoff oder Alkyl mit 1 bis 4 C-Atomen,

$R^6$ für die Gruppierungen $-CH=C=CH_2$ und $-C\equiv C-R^8$,

$R^7$ für geradkettig oder verzweigtes Alkyl mit 1 bis 4 C-Atomen, Alkenyl und Alkinyl mit 2 bis 4 C-Atomen, Alkoxyalkyl mit 1 bis 4 C-Atomen in jedem Alkylteil, gegebenenfalls substituiertes Phenyl oder Benzyl,

$R^8$ für Wasserstoff, Chlor, Brom oder Jod,

n für 1 oder 2

steht.

3. 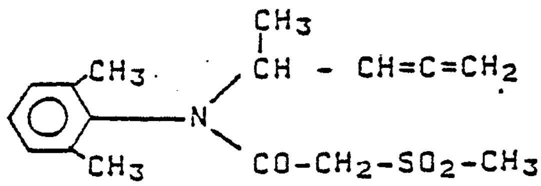

4. 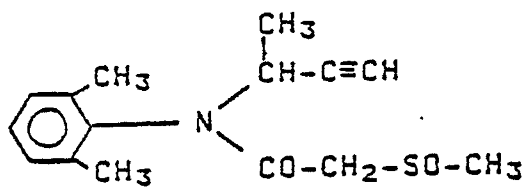

5. 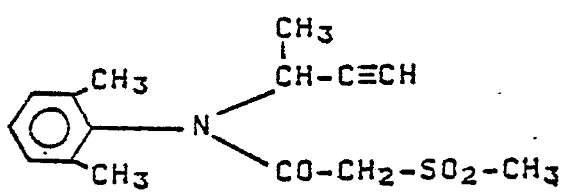

6. Verfahren zur Herstellung von Sulfinyl- und Sulfonyl-acetanilid-Derivaten der Formel

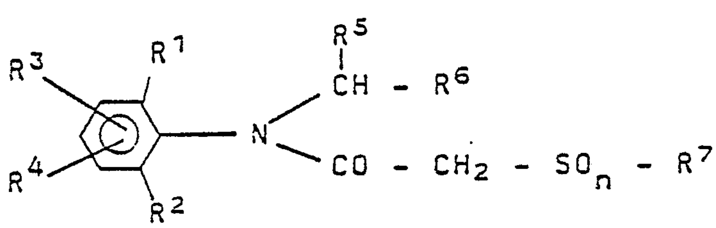

(I)

in welcher

$R^1$ für Alkyl, Alkoxy oder Halogen,

$R^2$ für Wasserstoff, Alkyl, Alkoxy oder Halogen,

$R^3$ und $R^4$ unabhängig voneinander für Wasserstoff, Alkyl oder Halogen,

$R^5$ für Wasserstoff oder Alkyl,

$R^6$ für die Gruppierungen $-CH=C=CH_2$ und $-C\equiv C-R^8$,

$R^7$ für Alkyl, Alkenyl, Alkinyl, Alkoxyalkyl, Aryl oder Aralkyl,

$R^8$ für Wasserstoff oder Halogen,

n für 1 oder 2

stehen, dadurch gekennzeichnet, daß

a) substituierte Acetanilide der Formel

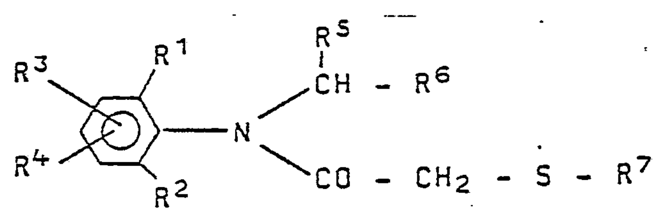

(II)

in welcher
$R^1$ bis $R^7$ die bei Formel I angegebene Bedeutung haben,

0070456

nach bekannten Methoden in üblicher Weise oxidiert, oder

b) Halogenacetanilide der Formel

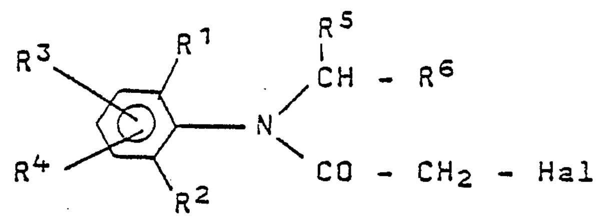

(III)

in welcher
$R^1$ bis $R^6$ die bei Formel I angegebene Bedeutung haben und
Hal für Chlor oder Brom steht,

mit Sulfen- bzw. Sulfinsäure-Derivaten der Formel

$$MSO_n - R^7 \quad \text{(IV)}$$

in welcher

M für Wasserstoff oder ein Alkalimetall steht und

$R^7$ und n die bei Formel I angegebene Bedeutung haben,

in Gegenwart eines Verdünnungsmittels und gegebenenfalls in Gegenwart eines Säurebinders umgesetzt oder

c) Aniline der Formel

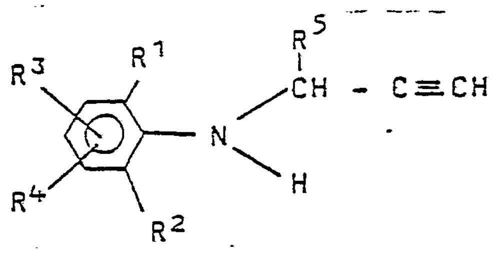

(V),

in welcher
$R^1$ bis $R^5$ die bei Formel I angegebene Bedeutung haben,

mit Säurehalogeniden oder -anhydriden der Formeln

$$Hal-CO-CH_2-SO_n-R^7 \qquad (VIa),$$

oder

$$O(CO-CH_2-SO_n-R^7)_2 \qquad (VIb),$$

in welchen
$R^7$ und n die bei Formel I angegebene Bedeutung haben, und

Hal für Chlor oder Brom steht,

in Gegenwart eines Verdünnungsmittels und gegebenenfalls in Gegenwart eines Säurebindemittels und/oder Katalysators umgesetzt, oder

d) erfindungsgemäße N-Propargyl-acetanilide der Formel

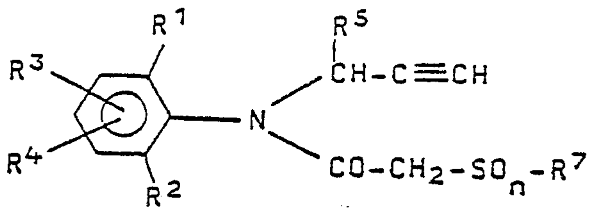

(VII)

in welcher
$R^1$ bis $R^5$, $R^7$ und n die bei Formel I angegebene Bedeutung haben,

in Gegenwart eines Katalysators mit Formaldehyd und Diisopropylamin umgesetzt, oder

e) erfindungsgemäße N-Propargyl-acetanilide der Formel

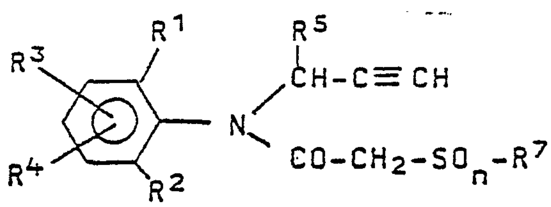

(VII)

in welcher
$R^1$ bis $R^5$, $R^7$ und n die bei Formel I angegebene Bedeutung haben,

mit Alkalihypohalogenit in Gegenwart eines Verdünnungsmittels umgesetzt werden.

7. Fungizide Mittel, gekennzeichnet durch einen Gehalt an mindestens einem Sulfinyl- und/oder Sulfonylacetanilid-Derivat der Formel I.

8. Verfahren zur Bekämpfung von Pilzen, dadurch gekennzeichnet, daß man Sulfinyl- und/oder Sulfonylacetanilid-Derivat der Formel I auf Pilze und ihren Lebensraum einwirken läßt.

9. Verwendung von Sulfinyl- und/oder Sulfonyl-acetanilid-Derivaten der Formel I zur Bekämpfung von Pilzen.

10. Verfahren zur Herstellung von fungiziden Mitteln, dadurch gekennzeichnet, daß man Sulfinyl- und/oder Sulfonyl-acetanilid-Derivate der Formel I mit Streckmitteln und/oder oberflächenaktiven Mitteln vermischt.

Europäisches Patentamt

# EUROPÄISCHER RECHERCHENBERICHT

0070456

Nummer der Anmeldung

EP 82 10 6057

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
| X | DE-A-2 362 743 (DIAMOND SHAMROCK) *Patentansprüche* | 1-10 | C 07 C 147/05<br>C 07 C 147/14<br>A 01 N 41/10 //<br>C 07 C 149/23 |
| A | GB-A-2 051 787 (F.HOFFMAN-LA ROCHE & CO) *Seite 1, Zeilen 5-27* | | |
| A | EP-A-0 010 673 (BAYER) *Patentansprüche* | | |
| P | EP-A-0 044 482 (BAYER AG) *Seite 2, Zeile 1 - Seite 3, Zeile 8* | | |
| | | | RECHERCHIERTE SACHGEBIETE (Int. Cl. 3) |
| | | | C 07 C 147/00 |

Der vorliegende Recherchenbericht wurde für alle Patentansprüche erstellt.

| Recherchenort | Abschlußdatum der Recherche | Prüfer |
|---|---|---|
| DEN HAAG | 20-10-1982 | PAUWELS G.R.A. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82